# EUROPÄISCHE PATENTANMELDUNG

(11) **EP 1 304 087 A2**
(43) Veröffentlichungstag der Anmeldung: **23.04.2003**
(21) Anmeldenummer: 02023468.8
(22) Anmeldetag: 21.10.2002
(51) Int. Cl.: A61C 8/00, A61B 17/16

(54) **Chirurgisches Werkzeug**

(30) Priorität: 22.10.2001 DE 10151355
(71) Anmelder: Dr.-medic-stom./UMF Temeschburg, Herbert Hatzlhoffer, 76337 Waldbronn (DE)
(72) Erfinder: Dr.-medic-stom./UMF Temeschburg, Herbert Hatzlhoffer, 76337 Waldbronn (DE)
(74) Vertreter: Blumenröhr, Dietrich

(57) **Zusammenfassung**

Bei der erfindungsgemäßen Vorrichtung handelt es sich um chirurgisches Werkzeug zur Präparation von Knochen, insbesondere für den Einsatz von Zahnimplantaten. Die Werkzeuge weisen ein rotierendes Bearbeitungsteil auf. Erfindungsgemäß wird das rotierende Bearbeitungsteil während des Bearbeitungsvorganges relativ zum Führungselement axial bewegt. Durch die Verschiebung des Bearbeitungsteils entlang des Führungselementes soll vermieden werden, dass das Bearbeitungsteil aus dem Bearbeitungskanal seitlich ausbricht. Weiterhin dient die erfindungsgemäße Vorrichtung dazu, dass der Bohrungsboden nicht vom Bearbeitungsteil durchstoßen wird.

## Beschreibung

Die Erfindung betrifft ein chirurgisches Werkzeug zur Präparation von Knochen, insbesondere für den Einsatz von Zahnimplantaten, mit einem rotierbaren Bearbeitungsteil.

Vor dem Einbringen von Implantaten in den Knochen findet zunächst eine Bearbeitung des Knochenmaterials mit speziellen chirurgischen Werkzeugen statt. Häufig wird zunächst eine Vorbohrung mit einem verhältnismäßig dünnen Bohrer vorgenommen bei der die Präparationstiefe durch Tiefenbegrenzungsinstrumente gesichert wird. Danach wird mit Hilfe eines sogenannten Formbohrers der Vorbohrungskanal aufgebohrt und erhält dadurch die für das Implantat notwendige Gestalt. In einem nächsten Schritt zur Präparation des Knochens wird die Formbohrung mit einem Gewinde versehen. Dazu wird ein Gewindeschneider in die Bohrung hineingedreht. Dabei kann es mit den herkömmlichen Bearbeitungswerkzeugen zu erheblichen Schwierigkeiten kommen. Es besteht stets die Gefahr, dass man den vorgegebenen Kanal während der Bearbeitung verlässt und umliegendes Knochenmaterial in Mitleidenschaft zieht. Dies gilt insbesondere bei der Präparation von Kieferknochen, weil sich die Implantatbohrung in das porös aufgebaute schwammartige Knochenmaterial der Spongiosa erstreckt und das Werkzeug unter Umständen in die Hohlräume des Knochengewebes eindringt. Dadurch wird der Kiefer des Patienten stark in Mitleidenschaft gezogen und gegebenenfalls sogar der Einsatz von Implantaten unmöglich.

Eine weitere Gefahr besteht darin, dass die Bearbeitung über die vorgegebene Tiefe der Bohrung hinausgeht und dabei den Bohrungs-Boden durchschlägt, wobei sowohl Kiefermaterial als auch Nerven in Mitleidenschaft gezogen werden.

Aufgabe der vorliegenden Erfindung ist es, chirurgische Werkzeuge zur Verfügung zu stellen, mit denen Knochenmaterial bearbeitet werden kann, ohne dass, dass das Werkzeug aus dem Bohrungskanal ausbricht bzw. den Bohrungskanal durchstößt.

Diese Aufgabe wird erfindungsgemäß dadurch gelöst, dass das Werkzeug ein Führungselement aufweist und das Bearbeitungsteil relativ zum Führungselement axial bewegbar ist.

Zu Beginn der Bearbeitung wird das Führungselement bzw. seine Spitze in den Vorbohrungskanal bzw. beim Gewindeschneiden in den noch zurückgebliebenen Rest des Vorbohrungskanals eingesetzt. Die rotierenden Bearbeitungsteile, z. B. Formbohrer oder Gewindeschneider arbeiten sich nun beim Bearbeiten des Knochens entlang des Führungselements in den Knochen. Hierbei werden sie definiert axial entlang des Führungselements verschoben und es besteht zu keinem Zeitpunkt die Gefahr, dass sie seitlich ausbrechen.

Eine besonders vorteilhafte Ausgestaltung der Erfindung besteht darin, dass auch die maximale Bearbeitungstiefe von dem Führungselement vorgebbar ist. Dadurch wird gewährleistet, dass die Bearbeitungsteile nicht über die vorgegebene Bohrung hinaus in den Knochen eindringen können. Der Bearbeitungsraum für das rotierbare Bearbeitungsteil wird exakt vorgegeben und es besteht weder die Gefahr eines seitlichen Ausweichens noch eines Überschreitens der Bohrungstiefe. Fehlerquellen bei der Bearbeitung werden also weitestgehend ausgeschlossen.

Die Tatsache, dass das Führungselement die maximale Arbeitstiefe vorgibt bringt viele Vorteile mit sich. So müssen nicht in umständlicher Weise sogenannte Tiefenstopringe oberhalb des Bohrers angebracht werden. Weiterhin erfolgt ein automatischer Bearbeitungsstop in der gewünschten Bearbeitungstiefe und es muss nicht an einer Längenskala die Eindringtiefe zuvor bzw. während des gesamten Bearbeitungsvorganges abgemessen werden. Durch das Führungselement kann eine maximale Bearbeitungstiefe auch dann vorgegeben werden, wenn das Bearbeitungsteil noch nicht vollständig in die Bohrung eingedrungen ist. Dies ist bei Verwendung von herkömmlichen Tiefenstopringen nicht möglich, da diese in der Regel oberhalb des Bearbeitungsteils befestigt werden müssen und dadurch gar nicht zum Anschlag an der Bohrungsmündung kämen.

Die maximale Bearbeitungstiefe kann entweder von dem Führungselement fertigungsgemäß vorgegeben sein oder bei Bedarf vom Benutzer entsprechend seinen Wünschen am Werkzeug selbst eingestellt werden. Die maximale Bearbeitungstiefe kann beliebig wählbar sein, so dass beispielsweise nur der obere Teil der Knochenbohrung bearbeitet wird oder die Bearbeitung fast bis zum Ende der Bohrung erfolgt.

Wenn die maximale Bearbeitungstiefe vom Führungselement vorgegeben wird, ist es beispielsweise denkbar, dass die Bearbeitungstiefe durch einen Anschlag vorgebbar ist. In diesem Fall kann das rotierbare Bearbeitungsteil nur bis zu diesem Anschlag axial am Führungselement verschoben werden. Eine Bearbeitung über den Anschlag hinaus ist dann nicht möglich.

Das Bearbeitungsteil kann sich um das Führungselement drehen, wobei das Führungselement ohne eigene Drehbewegungen in seiner Position verharrt und nur das Bearbeitungsteil Rotationsbewegungen ausführt.

In einer weiteren Ausführungsform des erfindungsgemäßen Werkzeugs dreht sich das Bearbeitungsteil mit dem Führungselement, wobei die Rotationsbewegung von dem Führungselement auf das Bearbeitungsteil oder umgekehrt übertragen werden kann.

Die Übertragung der Rotationsbewegung kann beispielsweise durch eine in Rotationsrichtung formschlüssige Verbindung erfolgen. Eine axiale Bewegung entlang des Führungselements kann in diesem Fall durch eine gegenseitige keilförmige oder konische Abschrägung der formschlüssigen Verbindung erzwungen werden. Aufgrund dieser Abschrägung wird das Bearbeitungsteil während des Arbeitsvorgangs in Richtung der Bohrung verschoben, da die Drehbewegung durch die Abschrägung teilweise in eine Bewegung mit axialer Komponente umgesetzt wird.

Eine Vorgabe der maximalen Bearbeitungstiefe ist auch dadurch möglich, dass die formschlüssige Verbindung zwischen dem Führungselement und dem Bearbeitungsteil ab einer bestimmten Tiefe lösbar ist und das Bearbeitungsteil in dieser Tiefe nicht mehr rotiert und dadurch im Knochen verharrt. In diesem Fall kommt es zu einem Hohldrehen des Führungselementes, wobei keine Rotationsbewegung mehr vom Führungselement auf das Bearbeitungsteil übertragen wird.

Es ist möglich, dass nach dem Verharren des Bearbeitungsteils im Knochen und dem Stoppen der Rotationsbewegung des Führungselements eine neue formschlüssige Verbindung mit dem Führungselement hergestellt und dadurch das Bearbeitungsteil aus dem Knochen entfernt wird. Beispielsweise kann hierbei das Bearbeitungsteil, zum Beispiel ein Gewindeschneider, bei einer Drehbewegung in entgegengesetzter Richtung wieder aus der Bohrung herausgedreht werden.

Für das erfindungsgemäße chirurgische Werkzeug können unterschiedliche Bearbeitungsteile eingesetzt werden. Als besonders wichtig haben sich zum einen Bohrer, insbesondere Formbohrer, und zum anderen Gewindeschneider bewährt.

Während der Bearbeitungsvorgänge wird Knochenmaterial abgetragen. Dieses Knochenmaterial sollte bereits während des Arbeitsvorgangs aus der Bohrung entfernt werden, da es sonst zu einem Verschmieren kommt. In der Mitte des Führungselements befindet sich hierzu eine Öffnung, durch die während des Bearbeitungsvorganges eine isotone Kochsalzlösung geleitet wird. Die wässrige Lösung dient zum einen der Kühlung des Bearbeitungswerkzeugs; zum anderen werden dadurch die abgetragenen Knochenteile aus der Bohrung im Knochen herausschwemmt. Um dies zu ermöglichen, wurden in einer vorteilhaften Ausführungsform der erfindungsgemäßen Vorrichtung die Führungselemente mit Spülungsnuten versehen. Die Kochsalzlösung tritt also an der Spitze des Führungselementes aus und läuft entlang der Spülungsnuten wieder aus der Bohrung im Knochen heraus. Auf ihrem Weg wird das Knochenmaterial mit ausgeschwämmt, so dass es nicht zum Verschmieren der Bohrung kommen kann. Ohne die Spülungsnuten ist es für die Kochsalzlösung äußerst schwierig, aus der Bohrung im Knochen zu entweichen, da das Führungselement in der Regel nur mit geringem Spiel in der Vorbohrung sitzt und es würde beim Durchleiten einer wässrigen Lösung gegebenenfalls ein Druck aufgebaut, der im ungünstigsten Fall dazu führen könnte, dass das Werkzeug aus der Bohrung herausgedrückt wird bzw. der dazu führen kann, dass der Kühlungskanal im Werkzeug nicht mehr durchströmt wird und es dadurch zu einer unerwünschten Erwärmung kommt. Eine Überhitzung des Werkzeugs kann erhebliche Schäden beim Patienten hervorrufen, da Knochenmaterial und darin eingebettete Nerven stark geschädigt werden.

Zweckmäßigerweise wird zwischen Bearbeitungsteil und Führungselement ein in Axialrichtung wirkendes Federelement vorgesehen, das das Führungselement aus dem Bearbeitungsteil herausdrückt. Vorteil der Verwendung der Federkraft zum Herausdrücken des Führungselementes besteht darin, dass das Werkzeug sofort zum Einsatz bereit steht. Das Führungselement muss nicht erst aus dem Werkzeug herausgezogen werden, um dann in die Bohrung eingeführt zu werden. Durch die Federkraft wird das Führungselement automatisch bis zu einem Anschlag herausgeführt und kann direkt in die Vorbohrung eingesetzt werden. Weiterhin besteht nicht die Gefahr, dass das Führungselement beispielsweise während des Bearbeitungsvorganges wieder zurück in das Werkzeug rutscht. Somit kann der Benutzer auch gegen die Schwerkraft arbeiten, ohne befürchten zu müssen, dass das Führungselement aus dem Eingriff mit der Vorbohrung gelangt und damit dessen Führungsfunktion gestört wird.

Weitere Merkmale und Vorteile der Erfindung ergeben sich aus der Beschreibung von Ausführungsbeispielen anhand der Zeichnung und aus den Zeichnungen selbst; dabei zeigt:
- Figur 1: einen axialen Halbschnitt durch eine erfindungsgemäße Bohrvorrichtung;
- Figur 2a: einen axialen Halbschnitt durch den Bohrkopf;
- Figur 2b: eine Vorderansicht des Bohrkopfs;
- Figur 3a: eine Seitenansicht des Führungselements der Bohrvorrichtung;
- Figur 3b: eine Vorderansicht des Führungselements;
- Figur 4: einen axialen Halbschnitt durch den Bohrkopfträger;
- Figur 5: einen axialen Halbschnitt durch eine erfindungsgemäße Gewindeschneidvorrichtung;
- Figur 6a: eine Seitenansicht mit teilweise axialem Halbschnitt durch das Führungselement der Gewindeschneidvorrichtung;
- Figur 6b: eine Vorderansicht des Führungselements;
- Figur 6c: einen Radialschnitt des Führungselementes entlang der Schnittebene A - A aus Figur 6a;
- Figur 7a: einen axialen Halbschnitt durch den Schneidkopf der Gewindeschneidvorrichtung;
- Figur 7b: eine Vorderansicht des Schneidkopfs;
- Figur 7c: eine Rückansicht des Schneidkopfs;
- Figur 8a: einen axialen Halbschnitt durch die Festlegungsmutter der Gewindeschneidvorrichtung;
- Figur 8b: eine Vorderansicht der Festlegungsmutter.

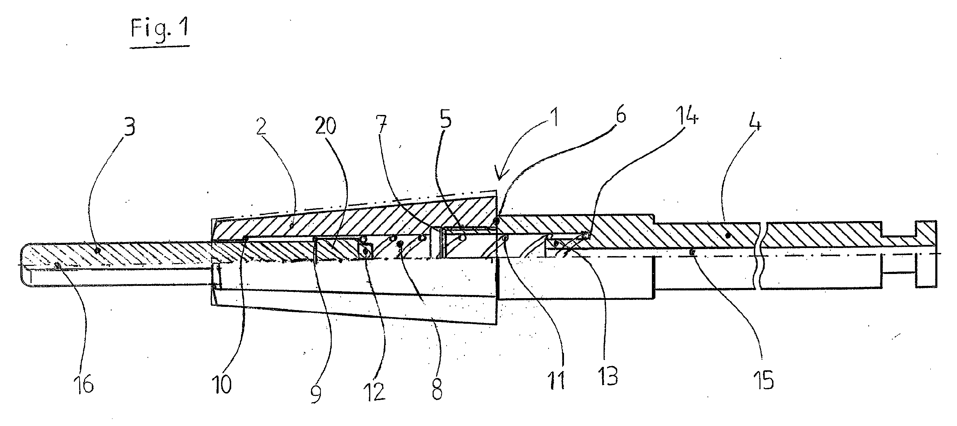

In Figur 1 ist eine Ausführung des erfindungsgemäßen Gegenstandes als Bohrvorrichtung 1 dargestellt. Die Bohrvorrichtung 1 besteht aus einem Bohrkopf 2, der sich als rotierendes Bearbeitungsteil um ein Führungselement 3 dreht. Der Bohrkopf ist auf einem Bohrkopfträger 4 befestigt. Der Bohrkopfträger 4 wird nach dem Einspannen in ein Winkelstück (nicht dargestellt) in Rotation versetzt und überträgt die Rotationsbewegung auf den Bohrkopf 2.

Bei dem Endstück des Bohrkopfträgers 4, das in das Winkelstück eingespannt wird, handelt es sich um ein DIN-genormtes Fertigungsteil. Der Bohrkopf 2 ist auf dem Bohrkopfträger 4 über ein Gewinde 5 festgelegt. Der Bohrkopf 5 kann auf den Bohrkopfträger 4 bis zu einem Anschlag 6 aufgeschraubt werden. Das Ende des Bohrkopfträgers 4 stößt dabei auch an den Anschlag 7 im Inneren des Bohrkopfs 2 an. In der Innenbohrung 8 des Bohrkopfs 2 ist das Führungselement 3 verschiebbar gelagert. Vor dem Einsatz des Werkzeugs liegt der Anschlag 9 des Führungselements 3 am Anschlag 10 des Bohrkopfs 2 an. Das Führungselement 3 wird dabei von einer Feder 11 an den Anschlag 10 gepresst.

Die Feder 11 sitzt an ihrem einen Ende auf dem Federsitz 12 des Führungselements 3 auf. An ihrem anderen Ende wird die Feder um den Federsitz 13 des Bohrkopfträgers 4 geführt. Die Feder 11 stößt sich dabei an der Innenwand 14 ab.

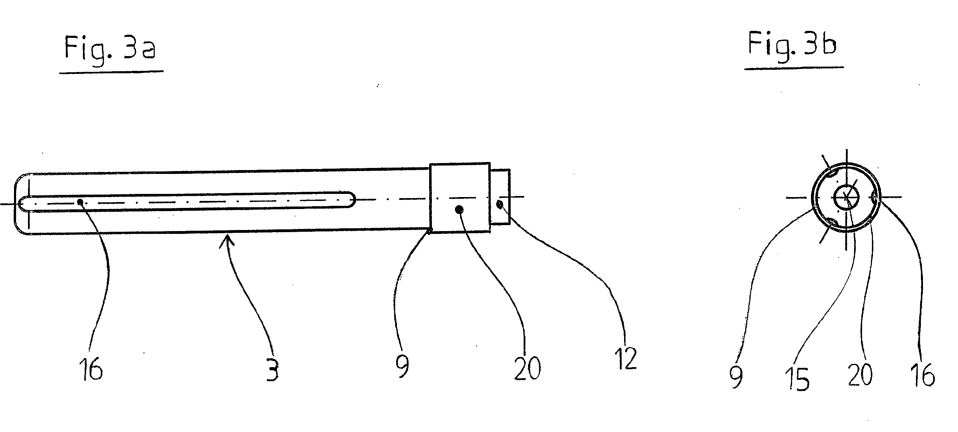

Nachdem eine Vorbohrung mit einem Bohrer vorgenommen wurde, die nur einen unwesentlich dickeren Durchmesser als das Führungselement 3 hat, wird das Führungselement 3 in diese Vorbohrung eingesetzt. Der Bohrkopf 2, der fest mit dem Bohrkopfträger 4 verbunden ist, wird in Rotation versetzt. Das Führungselement 3 dreht sich dabei nicht mit, sondern verharrt unbeweglich in der Vorbohrung. Durch Ausübung einer Kraft wird der rotierende Bohrkopf 2 in Richtung der Vorbohrung gedrückt. Dabei muss die von der Feder 11 aufgebrachte Gegenkraft überwunden werden. Die Feder staucht sich während dieses Vorgangs zusammen. Während des Bohrvorgangs verschiebt sich der Bohrkopf 2 solange entlang dem Führungselement 3, bis schließlich der Federsitz 13 des Bohrkopfsträgers 4 an den Federsitz 12 des Führungselements stößt. Durch diesen Anschlag wird die maximale Bearbeitungstiefe vorgegeben, bis zu der der rotierende Bohrkopf 2 in die Vorbohrung eindringen kann. Die Feder 11 wird bei diesem Vorgang ganz auf dem Federsitz 13 zusammengepresst. Durch das Innere des gesamten Werkzeuges läuft ein Kühlwasserkanal 15, siehe auch Figur 3b. Durch diesen Kanal 15 wird zur Kühlung des Werkzeugs und zum Herausspülen der abgetragenen Knochenteile eine isotone Kochsalzlösung geleitet. Die isotone Kochsalzlösung tritt an der Spitze des Führungselementes 3 während des Bearbeitungsvorganges aus und läuft dann entlang von Spülungsnuten 16 am Führungselement 3 zurück und durchspült hierbei den Bearbeitungsbereich des Bohrkopfes 2.

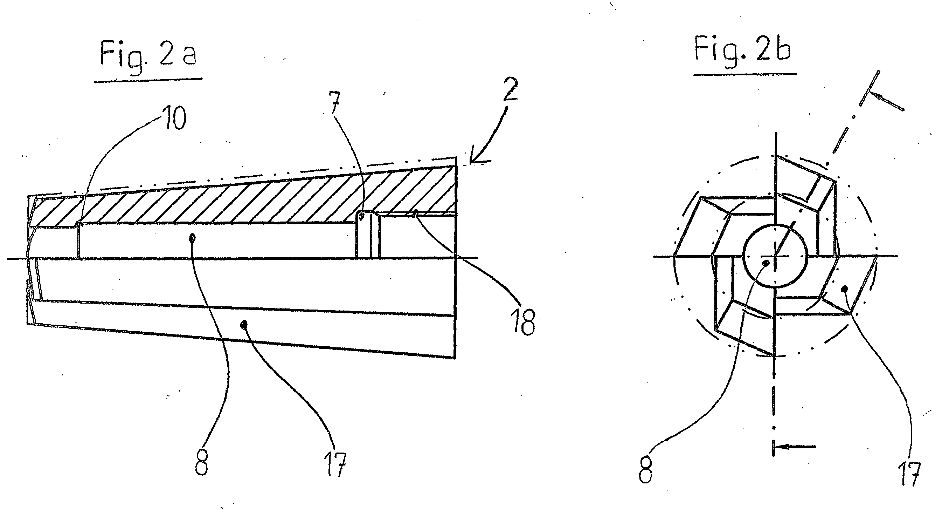

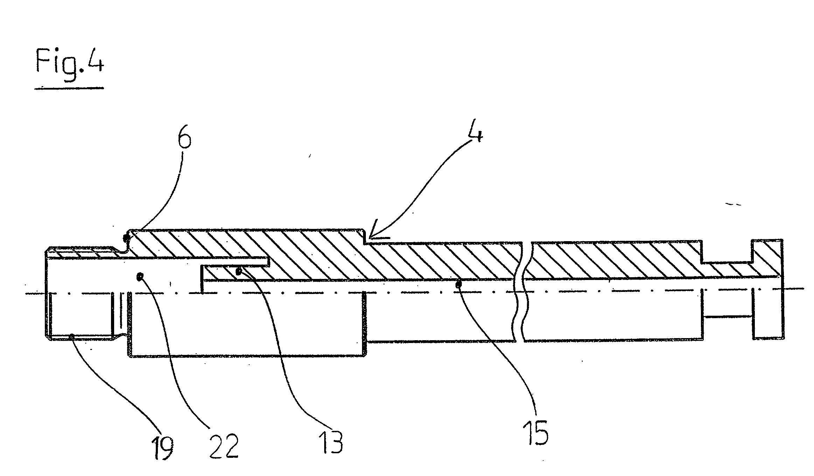

In Figur 2a ist ein axialer Halbschnitt des Bohrkopfs 2 dargestellt. Der Bohrkopf 2 hat insgesamt vier Schneiden 17 wie man aus der Figur 2b erkennt. Die Innenbohrung 8 des Bohrkopfs 2 weist insgesamt 3 Abstufungen auf. An dem den Bohrkopfträger zugewandten Ende hat die Bohrung den größten Durchmesser und ist mit einem Innengewinde 18 versehen. In dieses Innengewinde wird das Außengewinde 19, siehe Figur 4, des Bohrkopfträgers 4 bis zu dem Anschlag 7 eingeschraubt. Der Anschlag 7 wird durch eine Abstufung der Innenbohrung gebildet. Eine weitere Abstufung der Innenbohrung 8 bildet den Anschlag 10. An den Anschlag 10 wird vor dem Einsatz des chirurgischen Werkzeugs der Wulst 20, siehe Figur 3a und 3b, des Führungselements 3 durch die Feder 11 gedrückt.

In Figur 3a ist eine Seitenansicht des Führungselements 3 dargestellt. Die an der Spitze des Führungselements 3 austretende wässrige Lösung kann entlang der Spülungsnuten 16 zurückfließen. Die isotone Kochsalzlösung tritt durch die Öffnung des Kanals 15 aus. Der verdickte Wulst 20 des Führungselementes 3 bildet den Anschlag 9, der dazu dient, dass das Führungselement 3 nicht durch die Feder 11 vollständig aus dem Werkzeug herausgedrückt wird, sondern maximal bis zu dem Anschlag 10 des Bohrkopfes 2 wandern kann. An der dem Bohrkopfträger 4 zugewandten Seite des Führungselementes 3 befindet sich der Federsitz 12, dessen Vorderseite gleichzeitig als Anschlag für das Führungselement 3 dient. Das Führungselement 3 kann mit diesem Anschlag maximal bis an den Federsitz 13 des Bohrkopfträgers 4 verschoben werden. Dadurch ist auch die maximale Eindringtiefe des Bearbeitungselementes in die Bohrung vorgegeben. In Figur 3b ist eine Vorderansicht des Führungselementes 3 dargestellt. Man erkennt, dass im Ausführungsbeispiel insgesamt 3 Spülungsnuten 16 in das Führungselement hineingefräst wurden.

In Figur 4 ist der Bohrkopfträger 4 in einem axialen Halbschnitt dargestellt. Der Bohrkopfträger 4 wird mit seinem Außengewinde 19 in den Bohrkopf 2 bis zu seinem Anschlag 6 hineingeschraubt. An seiner dem Bohrkopf zugewandten Seite weist der Bohrkopfträger 4 eine Bohrung 22 auf, in der das Führungselement 3 bis zu dem Federsitz 13 verschoben werden kann. Bei dem vom Bohrkopf abgewandten Ende des Bohrkopfträgers 4 handelt es sich um ein DIN-genormtes Teil, das in ein Winkelstück eingespannt und in Rotation versetzt werden kann. In dem Bohrkopfträger 4 ist mittig eine Bohrung 15 zum Durchleiten von Kühl- bzw. Spülflüssigkeit durch das chirurgische Werkzeug eingebracht.

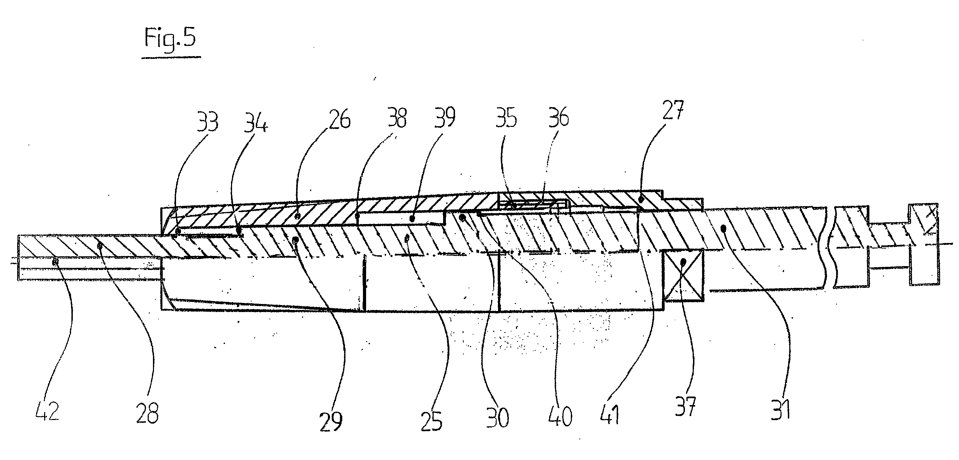

In Figur 5 ist eine Ausführung des erfindungsgemäßen chirurgischen Werkzeugs als Gewindeschneidvorrichtung in einem axialen Halbschnitt dargestellt. Die Gewindeschneidvorrichtung besteht aus einem Führungselement 25, einem Schneidkopf 26 und einer Festlegungsmutter 27.

Das Führungselement 25 lässt sich in die vier Hauptbereiche untergliedern Führungselementspitze 28, Mittelteil 29, Wulst 30 und Einspannteil 31.

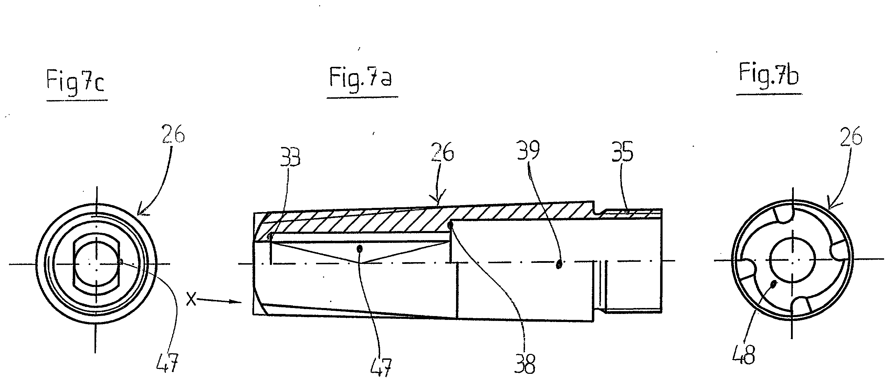

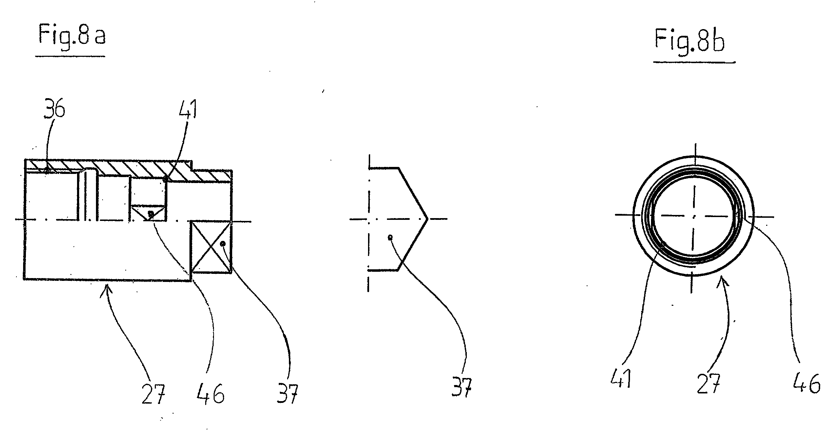

Der Schneidkopf 26 weist eine Innenbohrung mit insgesamt drei Abstufungen auf. Der Schneidkopf 26 wird vor Einsatz der Vorrichtung auf das Führungselement 25 geschoben. Der Anschlag 33 des Schneidkopfes 26 liegt dabei an dem Anschlag 34 des Führungselementes an. Der Schneidkopf 26 ist formschlüssig mit dem Führungselement 25 verbunden. Das Mittelteil 29 ragt bis zur Abstufung 33 in den Schneidkopf 26 hinein. Damit der Schneidkopf 26 nicht mehr vom Führungselement herunterrutschen kann, wird auf das Außengewinde 35 des Schneidkopfs das Innengewinde 36 der Festlegungsmutter 27 aufgeschraubt, siehe auch Figur 7a und 8a. Zum Festdrehen bzw. Lösen der Schraubverbindung kann gegebenenfalls ein Schraubschlüssel an dem Sechskant 37 der Festlegungsmutter 27 angesetzt werden.

Nachdem der Schneidkopf 26 bis zum Anschlag 34 auf das Führungselement 25 geschoben wurde und die Festlegungsmutter 27 am Schneidkopf aufgeschraubt wurde, wird die Führungselementspitze 28 in den nach der Formbohrung zurückgebliebenen Rest der Vorbohrung eingesetzt. Das Führungselement 25 wird über das Einspannteil 31 in Rotation versetzt. Durch das Mittelteil 29 wird die Rotationsbewegung aufgrund einer formschlüssigen Verbindung auf den Schneidkopf 26 und folglich auch auf die Festlegungsmutter 27 übertragen und der Schneidkopf 26 fängt an, ein Gewinde in den Knochen zu schneiden. Mit fortschreitendem Schneidvorgang wandert der Schneidkopf 26 mit samt der Festlegungsmutter 27 entlang dem Führungselement 25 in die Formbohrung hinein. Das axiale Hineinwandern in die Bohrung und damit das relative Verschieben des Führungselementes 25 und des Schneidkopfes 26 zueinander wird durch eine Abschrägung des Mittelteils 29 bewirkt.

Sobald der Schneidkopf 26 mit seiner Abstufung 38 über den Anschlag 34 des Führungselementes 25 hinausgewandert ist, löst sich die formschlüssige Verbindung zwischen Schneidkopf 26 und Führungselement 25. Der Schneidkopf 26 verharrt im Knochen. Das Führungselement 25 dreht hohl. Durch diese Konstruktion ist die Eindringtiefe des Schneidkopf 26 fest vorgegeben und es besteht daher nicht die Gefahr, dass der Boden der Bohrung durchstoßen wird.

Um den Schneidkopf 26 wieder aus der Bohrung zu entfernen, wird die Rotation des Führungselementes 25 gestoppt. Während der Schneidkopf 26 im Knochen verharrt, wird das Führungselement 25 aus der Bohrung so weit herausgeführt, dass der Anschlag 40 des Wulstes 30 an dem Anschlag 41 der Festlegungsmutter 27 anliegt. In dieser Stellung lässt sich dann der Wulst 30 formschlüssig in die Festlegungsmutter 27 einrasten. Das Führungselement 25 wird dann entgegen der Eindrehrichtung in Rotation versetzt. Über die formschlüssige Verbindung mit dem Wulst 30 dreht sich die Festlegungsmutter 27 mit und überträgt die Rotation auf den Schneidkopf 26, der wie bereits erwähnt über ein Gewinde mit der Festlegungsmutter 27 verbunden ist. Der Schneidkopf 26 wird entlang dem eingeschnittenen Gewinde aus der Bohrung wieder herausgedreht.

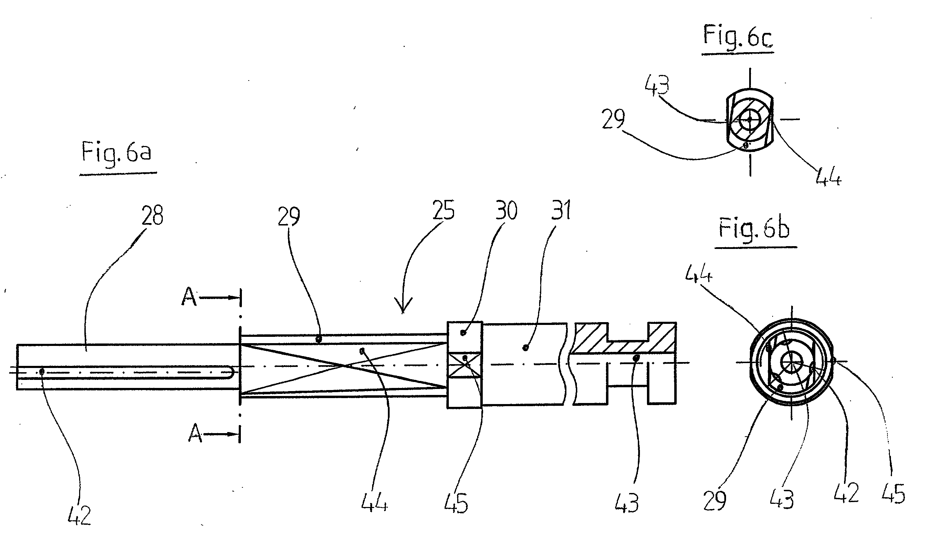

In Figur 6a ist eine Seitenansicht des Führungselements 25 dargestellt. Das Einspannteil 32 des Führungselements 25 ist als Halbaxialschnitt dargestellt. Die Führungselementspitze 28 ist mit Spülungsnuten 42 versehen. Entlang dieser Spülungsnuten 42 kann eine isotone Kochsalzlösung, die aus dem Kanal 43 austritt, zurückfließen. Die isotone Kochsalzlösung dient zur Kühlung des Werkzeugs und zum Ausspülen von abgetragenem Knochenmaterial. Der Kanal 43 ist mittig im Führungselement 25 eingebracht.

Wie man aus Figur 6a erkennt, ist das Mittelteil 29 an zwei einander gegenüberliegenden Seiten abgeflacht. Wie man ferner aus den Figuren 6b und 6c erkennt, sind diese abgeflachten Flächen 44 des Mittelteils 29 zur Führungselementspitze 28 hin abgeschrägt. Dieses Abschrägen dient dazu, dass der mit dem Mittelteil 29 formschlüssig verbundene Schneidkopf 26 während des Schneidvorgangs entlang dem Führungselement 25 in die Bohrung hineinverschoben wird.

In Figur 6a erkennt man den Aufbau des Wulstes 30, der ebenfalls zwei einander gegenüberliegende abgeflachte Seitenwände 45 aufweist. In die abgeflachten Flächen 45 werden zum Herausdrehen die abgeflachten Innenflächen 46 der Festlegungsmutter 27 eingerastet.

In Figur 6b ist eine Ansicht des Führungselementes von vorne dargestellt. In dieser Ansicht erkennt man deutlich die beiden abgeflachten Seitenflächen 45 des Wulstes 30.

In Figur 6c ist ein Schnitt entlang der Schnittebene A-A eingezeichnet. Man erkennt einen Kanal 43, der sich durch das gesamte Führungselement 25 erstreckt und durch den die isotone Salzlösung geleitet wird. Weiterhin wird bei diesem Schnitt deutlich, dass das Mittelteil 29 zur Führungselementspitze 28 hin abgeschrägt ist. Weiterhin erkennt man deutlich die beiden abgeflachten Seitenflächen 44 des Mittelteils 29.

In Figur 7a ist der Schneidkopf 26 im axialen Halbschnitt dargestellt. Man erkennt, dass die Bohrung im Inneren des Schneidkopfs 26 insgesamt drei Abstufungen hat. Durch den kleinsten Durchmesser der Innenbohrung wird die Führungselementspitze 28 geführt. Der mittlere Teil der Innenbohrung dient zur formschlüssigen Verbindung mit dem Mittelteil 29 des Führungselements 25. Dazu weist die Innenbohrung in diesem Bereich zwei einander gegenüberliegende abgeflachte Innenwände auf, durch die eine formschlüssige Verbindung mit dem Mittelteil 29 hergestellt werden kann.

In Figur 7b sind die Schneidzähne 48 des Schneidkopfes 26 dargestellt, die dazu dienen, ein Gewinde in den Knochen zu schneiden.

In Figur 7c ist eine Ansicht des Schneidkopfs 26 von hinten dargestellt. Man erkennt die abgeflachten Innenwände 47 im Inneren des Schneidkopfs 26.

In Figur 8a ist die Festlegungsmutter 27 als axialer Halbschnitt dargestellt. Man erkennt die abgeflachten Seitenwände 46, die beim Herausdrehen formschlüssig in die abgeflachten Seitenwände des Wulstes 30 eingerastet werden können. Zur Drehung der Festlegungsmutter 27 kann an dem Sechskant 37 ein Schraubenschlüssel angesetzt werden.

In Figur 8b ist eine Vorderansicht der Festlegungsmutter 27 dargestellt. Man erkennt in dieser Darstellung ebenfalls die seitlich abgeflachten Innenflächen 46, die beim Herausdrehen in die Außenflächen des Wulstes 30 einrasten.

Zusammenfassend zeichnet sich das erfindungsgemäße chirurgische Werkzeug dadurch aus, dass der Benutzer sobald einmal die Vorbohrung gesetzt ist, die darauf folgenden Arbeitsschritte ohne große Fehlerquellen durchführen kann. Mit dem erfindungsgemäßen Werkzeug besteht weder die Gefahr eines seitlichen Ausbrechens aus der Bohrung noch eines Durchschlagens der Bohrung nach unten. Dies wird dadurch gewährleistet, dass das Werkzeug ein Führungselement aufweist und das rotierende Bearbeitungsteil während des Bearbeitungsvorganges axial am Führungselement verschoben wird. Mit dem erfindungsgemäßen Werkzeug ist es auch für Anfänger möglich, die notwendigen Bearbeitungsschritte zur Präparation von Knochen, insbesondere für den Einsatz von Zahnimplantaten, durchzuführen.

Abschließend sei angemerkt, dass das erfindungsgemäße Werkzeug in allen Bereichen der Knochenchirurgie eingesetzt werden kann. Es ist keinesfalls nur auf die Kieferchirurgie beschränkt. Das erfindungsgemäße Prinzip lässt sich weiterhin auf alle Bearbeitungsteile übertragen, die im Rahmen der Bearbeitung von Knochenmaterial eingesetzt werden.

## Patentansprüche

1. Chirurgisches Werkzeug zur Präparation von Knochen, insbesondere für den Einsatz von Zahnimplantaten, mit einem rotierbaren Bearbeitungsteil (2, 26),
**dadurch gekennzeichnet,**
**dass** das Werkzeug ein Führungselement (3, 25) aufweist, und dass das Bearbeitungsteil (2, 26) relativ zum Führungselement (2, 35) axial bewegbar ist.

2. Chirurgisches Werkzeug nach Anspruch 1,
**dadurch gekennzeichnet,**
**dass** eine maximale Bearbeitungstiefe von dem Führungselement (3, 25) vorgebbar ist.

3. Chirurgisches Werkzeug nach Anspruch 2,
**dadurch gekennzeichnet,**
**dass** die Bearbeitungstiefe durch einen Anschlag (13) vorgebbar ist.

4. Chirurgisches Werkzeug nach Anspruch 1,
**dadurch gekennzeichnet,**
**dass** das Bearbeitungsteil (2) um das Führungselement (3) rotierbar ist.

5. Chirurgisches Werkzeug nach Anspruch 1,
**dadurch gekennzeichnet,**
**dass** das Bearbeitungsteil (26) mit dem Führungselement (25) rotierbar ist.

6. Chirurgisches Werkzeug nach Anspruch 1,
**dadurch gekennzeichnet,**
**dass** die Rotationsbewegung durch eine formschlüssige Verbindung von dem Führungselement (25) auf das Bearbeitungsteil (26) übertragbar ist.

7. Chirurgisches Werkzeug nach Anspruch 6,
**dadurch gekennzeichnet,**
**dass** das Bearbeitungsteil (26) durch eine Abschrägung der formschlüssigen Verbindung relativ zum Führungselement (25) axial bewegbar ist.

8. Chirurgisches Werkzeug nach Anspruch 6,
**dadurch gekennzeichnet,**
**dass** die formschlüssige Verbindung zwischen Führungselement (25) und Bearbeitungsteil (26) ab einer bestimmten Bearbeitungstiefe lösbar ist, so dass das Bearbeitungsteil (26) in dieser Tiefe im Knochen verharrt.

9. Chirurgisches Werkzeug nach Anspruch 8,
**dadurch gekennzeichnet,**
**dass** nach dem Verharren des Bearbeitungsteils (26) eine neue formschlüssige Verbindung mit dem Führungselement (25) herstellbar ist und dadurch das Bearbeitungsteil (26) aus dem Knochen entfernbar ist.

10. Chirurgisches Werkzeug nach Anspruch 1,
**dadurch gekennzeichnet**
**dass** es sich bei dem Bearbeitungsteil um einen Gewindeschneider (26) handelt.

11. Chirurgisches Werkzeug nach Anspruch 1,
**dadurch gekennzeichnet,**
**dass** es sich bei dem Bearbeitungsteil um einen Bohrer (2) handelt.

12. Chirurgisches Werkzeug nach Anspruch 1,
**dadurch gekennzeichnet,**
**dass** das Führungselement (3, 25) Spülungsnuten (16, 42) aufweist.

13. Chirurgisches Werkzeug nach Anspruch 1,
**dadurch gekennzeichnet,**
**dass** das Bearbeitungsteil (2) gegen eine Rückstellkraft, insbesondere eine Federkraft, relativ zum Führungselement (3) axial bewegbar ist.
